# DEMANDE DE BREVET EUROPEEN

(11) **EP 1 473 233 A1**
(43) Date de publication de la demande: **03.11.2004**
(21) Numéro de dépôt: 04291059.6
(22) Date de dépôt: 23.04.2004
(51) Int. Cl.: B65B 31/00, B65B 3/12

(54) **Procédé et ensemble de remplissage d' un récipient de produit fluide ainsi que le réservoir du produit fluide**

(30) Priorité: 28.04.2003 FR 0305158
(71) Demandeur: VALOIS S.A.S., 27110 Le Neubourg (FR)
(72) Inventeur: Garcia, Firmin, 27000 Evreux (FR); Lecoutre, Jean-Paul, 27160 Breteuil sur Iton (FR)
(74) Mandataire: CAPRI

(57) **Abrégé**

La présente invention propose un ensemble et un procédé de remplissage de réservoir de produit fluide sur lequel est monté un organe de distribution. Une source externe de produit fluide est connectée à l'organe de distribution, et le produit fluide de la source est aspiré dans le réservoir vide préalablement vidé par l'organe de distribution par la dépression qui règne dans le réservoir.

## Description

La présente invention concerne un procédé, une source externe de remplissage ainsi qu'un ensemble de remplissage de réservoir de produit fluide, liquide, pâteux ou même pulvérulent. L'ensemble de distribution comprend un distributeur de produit fluide essentiellement constitué d'un réservoir de produit fluide destiné à contenir du produit fluide et un organe de distribution, tel qu'une pompe montée dans une ouverture du réservoir de produit fluide pour pouvoir y prélever du produit fluide et le distribuer à travers une tête de distribution qui présente avantageusement un orifice de distribution où le produit fluide peut être recueilli ou pulvérisé. Ce genre de distributeur de produit fluide est fréquemment utilisé dans les domaines de la parfumerie, de la cosmétique ou encore de la pharmacie. Il s'agit de distributeurs manuels que l'utilisateur peut saisir à l'aide d'une main au niveau du réservoir du distributeur et peut appuyer, par exemple à l'aide de l'index, sur un poussoir permettant d'actionner la pompe et ainsi obtenir une distribution de produit fluide.

La présente invention s'applique plus particulièrement au distributeur présentant un réservoir rigide dont le volume utile ne varie pas. Le réservoir peut être réalisé en matière plastique, en verre ou encore en métal. L'organe de distribution, à savoir la pompe, est généralement fixé sur le réservoir au moyen d'une bague de fixation. A cet effet, le réservoir forme généralement un col définissant une ouverture dans laquelle est fixée la pompe. La présente invention s'applique également plus particulièrement à un certain type de pompe, fréquemment désigné sous l'appellation « airless », qui fonctionne sans reprise d'air, c'est-à-dire que le volume de produit fluide prélevé par la pompe dans le réservoir indéformable n'est pas remplacé par un volume équivalent d'air extérieur. Par conséquent, à chaque actionnement de la pompe, la pression diminue à l'intérieur du réservoir pour atteindre un maximum de dépression lorsque le réservoir est vide de produit fluide.

En général, lorsque ce type particulier de distributeur « airless » est vide, il est destiné au rebut.

Le but de la présente invention est de définir un procédé de remplissage de ce type de distributeur sans avoir à retirer la pompe du réservoir. L'invention a également pour but de définir une source de remplissage ainsi qu'un ensemble de distribution et de remplissage permettant de remplir ce type de distributeur sans avoir à retirer la pompe.

Pour ce faire, la présente invention propose un procédé comprenant :
- une étape préalable de vidage du réservoir par prélèvement du produit fluide au moyen de la pompe, de sorte qu'une dépression est créée dans le réservoir,
- une étape d'alimentation externe en produit fluide consistant à alimenter le canal de sortie avec du produit fluide issu d'une source externe, et
- une étape d'ouverture consistant à ouvrir le clapet de sortie, en maintenant le clapet d'entrée ouvert, de sorte que la dépression régnant dans le réservoir aspire du produit fluide issu de la source à travers le canal de sortie, le clapet de sortie ouvert, la chambre de pompe et le clapet d'entrée ouvert jusque dans le réservoir.

Avantageusement, le produit fluide issu de la source externe est alimenté au canal de sortie à une pression sensiblement égale ou inférieure à la pression atmosphérique. Ainsi, le procédé selon l'invention consiste à remplir un réservoir de distributeur en dépression par aspiration de produit fluide à travers la pompe en ouvrant le clapet de sortie et d'entrée.

L'invention propose également une source externe de remplissage de produit fluide pour un distributeur de produit fluide, ledit distributeur comprenant :
■ un réservoir destiné à contenir un produit fluide à une pression sensiblement égale ou inférieure à la pression atmosphérique,
■ une pompe associée de manière étanche au réservoir pour prélever du produit fluide dans le réservoir, la pompe étant du type sans reprise d'air de sorte que la pression à l'intérieur du réservoir diminue à mesure que la pompe prélève du produit fluide, ladite pompe comprenant un clapet d'entrée et un clapet de sortie, le clapet d'entrée communiquant avec le réservoir et le clapet de sortie communiquant avec un canal de sortie, le clapet d'entrée communiquant avec le clapet de sortie à travers une chambre de pompe,
   la source externe de remplissage en produit fluide comprenant :

■ une réserve de produit fluide,
■ un orifice d'alimentation en produit fluide destiné à être connecté au canal de sortie du distributeur,
■ un clapet d'alimentation disposé entre la réserve et l'orifice d'alimentation, ledit clapet d'alimentation étant sollicité en position ouverte lorsque l'orifice d'alimentation est connecté au canal de sortie du distributeur, ledit clapet d'alimentation comprenant un organe mobile en appui étanche sélectif sur un siège de clapet. Avantageusement, le produit fluide contenu dans la réserve de la source est à une pression sensiblement égale ou légèrement inférieure à la pression atmosphérique, de sorte que le produit fluide issu de l'orifice d'alimentation est aspiré dans le réservoir du distributeur par la dépression qui y règne.

Selon un autre aspect de l'invention, la source comprend des moyens d'ouverture aptes à ouvrir le clapet de sortie du distributeur. Avantageusement, les moyens d'ouverture coopèrent également avec le clapet d'alimentation pour l'ouvrir avant d'ouvrir le clapet de sortie. De préférence, les moyens d'ouverture comprennent une broche définissant une extrémité de liaison solidaire de l'organe mobile et une extrémité libre de poussée destinée à être engagée dans le canal de sortie du distributeur pour ouvrir le clapet de sortie. Il est préférable que la broche ouvre d'abord le clapet d'alimentation pour permettre au fluide issu de la réserve de produit fluide de parvenir dans le canal de sortie jusqu'au niveau du clapet de sortie. Ensuite, le clapet de sortie peut être ouvert de manière à aspirer le produit fluide issu de la réserve à travers le clapet d'alimentation ouvert. Si le clapet de sortie venait à être ouvert en premier, la dépression plaquerait le clapet d'alimentation en position fermée.

Selon une autre caractéristique intéressante de l'invention, le canal de sortie du distributeur est formé par une tige d'actionnement déplaçable axialement dans un corps de pompe, la tige supportant un piston apte à faire varier le volume utile de la chambre de pompe, la source externe comprenant alors des moyens de blocage pour bloquer la tige d'actionnement par rapport au corps de pompe. Avantageusement, les moyens de blocage comprennent au moins deux mâchoires articulées entre lesquelles la tige d'actionnement est destinée à être maintenue de manière serrante. De préférence, les moyens de blocage comprennent un système de levier apte à exercer une traction qui sollicite la tige hors du corps alors qu'elle est déjà maintenue par les mâchoires. Dans un mode de réalisation pratique, les moyens de blocage comprennent au moins deux bras articulés présentant chacun une extrémité fixe et une extrémité libre formant une mâchoire, les mâchoires étant disposées de manière à définir un logement central de taille variable à mesure que les mâchoires se déplacent par pivotement des bras, les bras formant en outre chacun une surface d'appui destinée à venir en contact d'appui sur un élément fixe du distributeur, un pivotement des bras dans le sens d'une diminution de la taille du logement central, alors que la tige est insérée dans ce logement, amène les mâchoires en prise serrante avec la tige et les surfaces d'appui en contact de l'élément fixe de manière à exercer une traction sur la tige hors du corps par l'intermédiaire des mâchoires. Il a été démontré en pratique qu'il est pratiquement indispensable de maintenir la tige d'actionnement lors de l'ouverture du clapet de sortie par la broche. En effet, l'ouverture du clapet de sortie a tendance à déplacer la tige d'actionnement vers l'intérieur du corps de pompe sous l'effet de la dépression qui règne dans le réservoir. De plus, le système de levier permet de remonter très légèrement la tige d'actionnement hors du corps de pompe, ce qui augmente le passage d'écoulement du fluide au niveau des clapets d'entrée et de sortie ouverts.

Selon un aspect pratique de l'invention, la source externe peut être constituée par un autre distributeur de produit fluide comprenant également un réservoir et une pompe. La source peut par exemple se présenter sous la forme d'un distributeur présentant un réservoir de moyenne à grande capacité dont le fond est équipé des moyens d'ouverture et de blocage. Le distributeur à remplir peut avoir un réservoir de faible capacité qu'il est nécessaire de recharger fréquemment. L'ensemble de distribution et de remplissage permet, pour un produit fluide identique, de disposer d'un distributeur domestique de grande capacité et d'un distributeur de voyage de faible capacité.

Selon une forme de réalisation pratique, le clapet d'entrée comprend un siège de clapet et un organe mobile, le clapet de sortie comprend un siège de clapet et un organe mobile, les organes mobiles étant formés par une pièce de clapet de sorte que les organes mobiles sont solidaires en déplacement, les clapets d'entrée et de sortie peuvent ainsi être ouverts simultanément. Ce type de pompe permet de vider entièrement un réservoir de distributeur sans reprise d'air en créant une dépression relativement importante. Cette dépression servira au remplissage à l'aide de la source externe.

L'invention a également pour objet un ensemble de distribution et de remplissage comprenant un distributeur de produit fluide comprenant un réservoir destiné à contenir un produit fluide à une pression sensiblement égale ou inférieure à la pression atmosphérique, une pompe associée de manière étanche au réservoir pour prélever du produit fluide dans le réservoir, la pompe étant du type sans reprise d'air de sorte que la pression à l'intérieur du réservoir diminue à mesure que la pompe prélève du produit fluide, ladite pompe comprenant un clapet d'entrée et un clapet de sortie, le clapet d'entrée communiquant avec le réservoir et le clapet de sortie communiquant avec un canal de sortie, le clapet d'entrée communiquant avec le clapet de sortie à travers une chambre de pompe, et une source externe de remplissage telle que définie ci-dessus.

L'invention sera maintenant plus amplement décrite en référence aux dessins joints donnant à titre d'exemple non limitatif un mode de réalisation de l'invention.

Sur les figures :
- la figure 1 est une vue en section transversale verticale à travers un ensemble de distribution et de remplissage de produit fluide selon l'invention,
- les figures 2a et 2b sont des vues respectivement agrandies et fortement agrandies d'une partie de l'ensemble de la figure 1 en position de repos,
- les figures 3a et 3b sont des vues similaires aux figures 2a et 2b juste avant l'étape de remplissage,

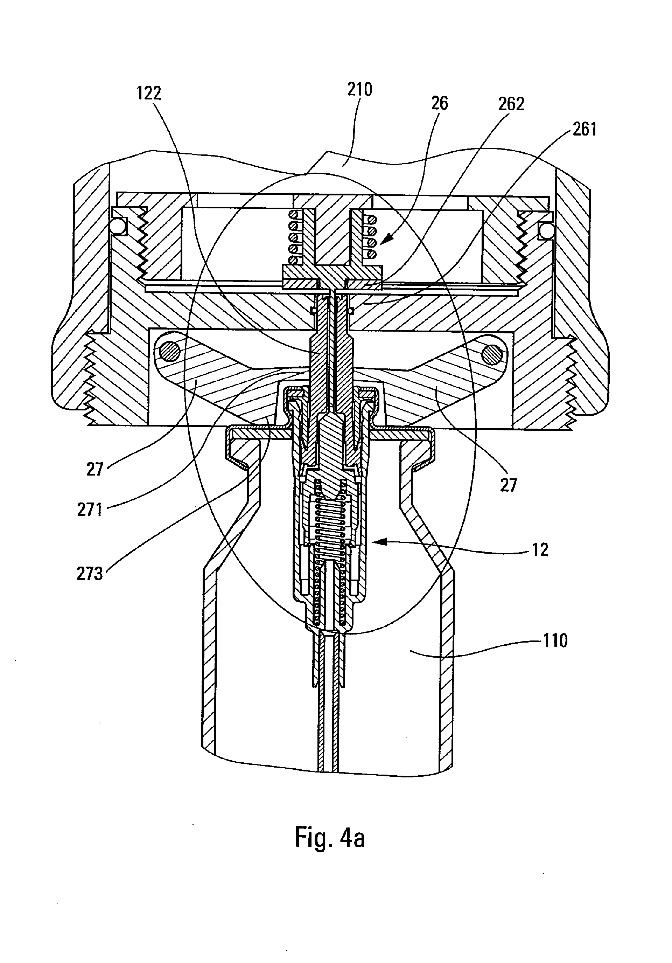

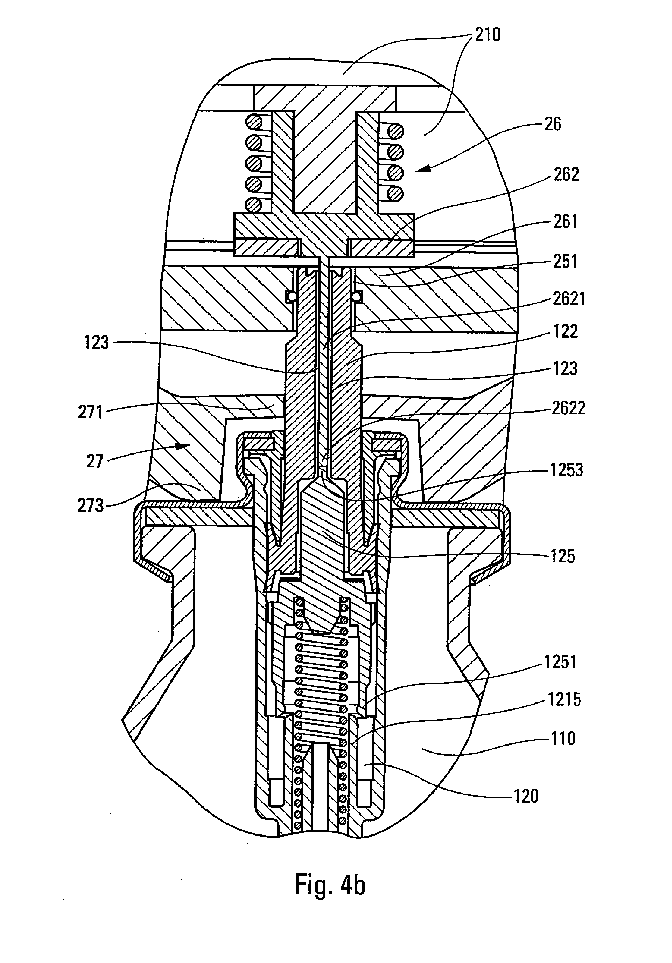

- les figures 4a et 4b sont des vues similaires aux figures 2a et 2b en cours de remplissage.

Le procédé et la source de remplissage de réservoir selon l'invention s'applique à un distributeur de produit fluide particulier qui va d'abord être décrit en détail. Ce distributeur, visible sur la plupart des figures, est désigné dans son ensemble par la référence numérique 1.

Le distributeur de produit fluide 1 comprend essentiellement un récipient 11 et un organe de distribution 12, qui est ici une pompe. D'ailleurs, dans la suite de la description, nous ferons toujours référence à une pompe en tant qu'organe de distribution. Le récipient 11 est un récipient à paroi rigide, qui peut être réalisé en verre, en matière plastique, en métal ou en tout autre matériau rigide. Le récipient comprend un fond, une paroi latérale ainsi qu'un col 111 qui définit une ouverture donnant accès à l'intérieur du récipient. Le volume interne définit à l'intérieur du récipient sert de réservoir de produit fluide 110 destiné à contenir un produit fluide, tel que du parfum, une lotion, ou plus généralement tous produits fluides de parfumerie, de cosmétique ou encore de pharmacie. Etant donné que le récipient 11 est rigide, le réservoir de produit fluide 110 définit une contenance fixe déterminée.

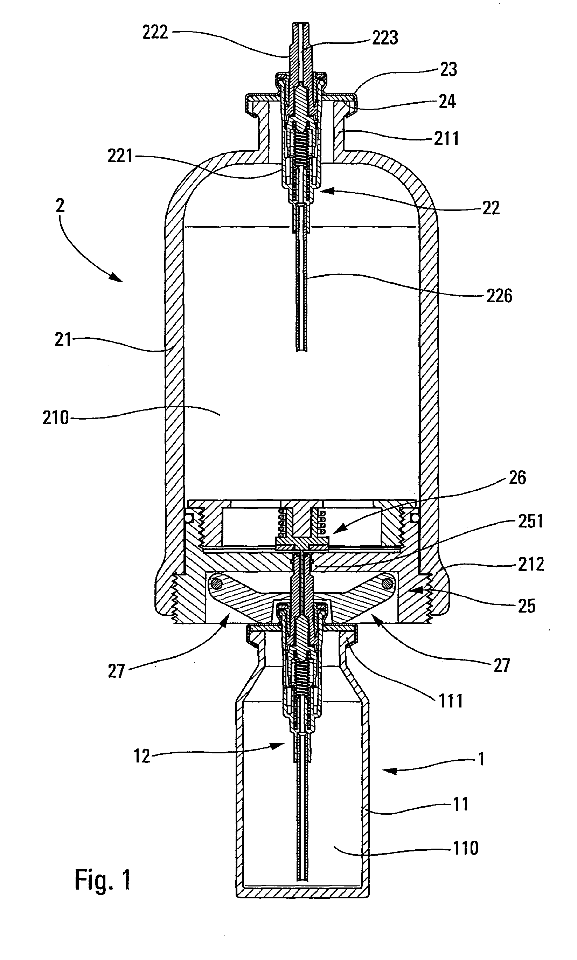

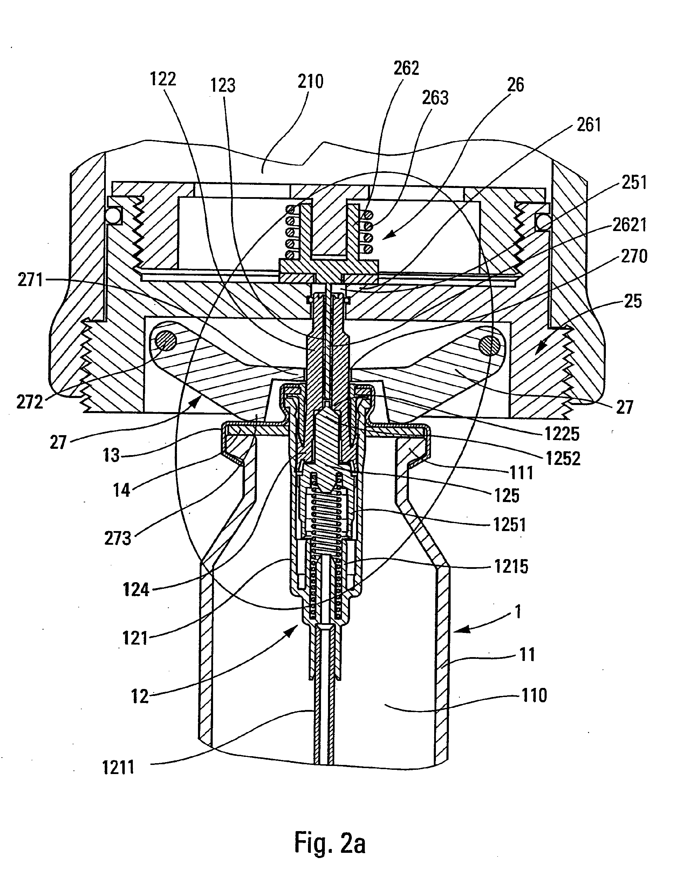

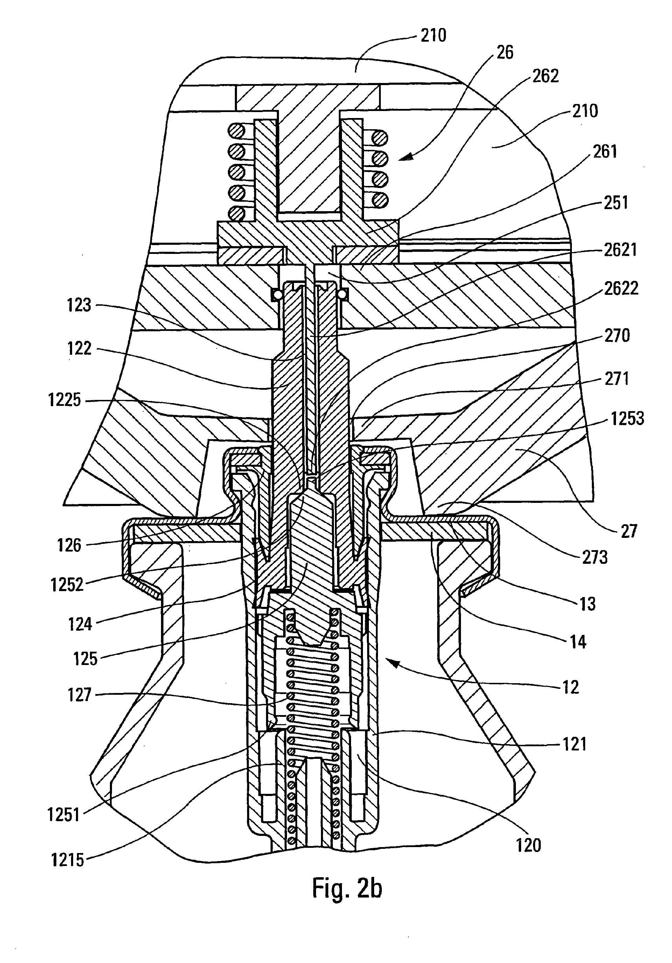

La pompe 12, visible sur la figure 1, et bien plus clairement sur les figures 2a et 2b, est une pompe manuelle classique à précompression. La pompe est du type « airless » c'est-à-dire sans reprise d'air de sorte que la quantité de produit qu'elle prélève n'est pas remplacée par un volume correspondant d'air extérieur. Nous verrons ci-après les conséquences au niveau du distributeur et plus particulièrement au niveau du réservoir 110. La pompe 12 comprend un corps de pompe 121 définissant une entrée avantageusement pourvue d'un tube plongeur 1211. Le corps de pompe 121 définit intérieurement un fût de coulissement. Un piston 124 est monté coulissant dans ce fût. Le piston 124 est ici solidaire d'une tige d'actionnement 122 formant intérieurement un canal de sortie 123. Le piston 124 et la tige d'actionnement 122 sont ici réalisés de manière monobloc. La tige d'actionnement 122 forme ici également un siège de clapet de sortie 1225 situé à l'extrémité inférieure du canal de sortie 123. La pompe comprend d'autre part une pièce de clapet 125 engagée dans le corps de pompe 121. La pièce de clapet 125 est sollicitée contre la tige d'actionnement 122 par un ressort de rappel et de précompression 127. Le ressort 127 agit de manière à pousser la pièce de clapet 125 et la tige d'actionnement 122 hors du corps de pompe 121 par son extrémité supérieure opposée à l'entrée. Pour maintenir la tige d'actionnement 122 et la pièce de clapet 125 à l'intérieur du corps de pompe, il est prévu une virole 126 engagée en force dans le corps de pompe et servant de butée pour la tige d'actionnement et la pièce de clapet en position de repose de la pompe. La virole 126 peut par exemple venir en butée contre le piston 124. La pièce de clapet 125 définit deux organes mobiles de clapet, à savoir un organe mobile de clapet de sortie 1252 et un organe mobile de clapet d'entrée 1251. La pièce de clapet 125 forme un pointeau définissant une surface tronconique 1252 destinée à venir en contact étanche avec le siège de clapet 1225 formé par la tige d'actionnement 122. La surface tronconique 1252 est sollicitée contre le siège 1225 par la force exercée par le ressort 127. La surface tronconique 1252 se prolonge pour former un sommet 1253 engagé à l'intérieur du canal de sortie 123 au niveau de son extrémité inférieure. La pièce de clapet 125 forme également une jupe dont l'extrémité libre forme un cordon 1251 destiné à venir en contact coulissant étanche à l'extérieur d'une tubulure 1215 formée par le corps de pompe 121 sensiblement au niveau de son entrée. Le ressort 127 est engagé à l'intérieur de cette tubulure 1215. Cette tubulure 1215 fait office de siège de clapet d'entrée alors que le cordon formé à l'extrémité inférieure de la jupe forme l'organe mobile de clapet d'entrée 1251. En position de repos représenté sur les figures 2a et 2b, le ressort 127, qui prend appui sur la pièce de clapet 125 sollicite la pièce de clapet 125 contre la tige d'actionnement 122 qui elle-même est sollicitée contre la virole 126 engagée en force dans le corps de pompe 121. Le clapet de sortie formé par le siège 1225 et l'organe mobile 1252 est fermé alors que le clapet d'entrée formé par le cordon 1251 et la tubulure 1215 est ouvert. En appuyant sur la tige d'actionnement 122 de manière à l'enfoncer dans le corps de pompe 121, le piston 124 coulisse de manière étanche dans le fût du corps et pousse la pièce de clapet 125 vers le bas contre l'action du ressort 127. Il est aisé de comprendre qu'après une course faible, le cordon 1251 va venir en prise étanche autour de la tubulure 1215. Un volume est alors isolé de l'extérieur par le clapet de sortie fermé et de l'intérieur du réservoir 110 par le clapet d'entrée fermé. Ce volume constitue une chambre de pompe 120. En continuant à appuyer sur la tige 122, le piston 124 continue à descendre dans le fût du corps, ce qui résulte en une diminution du volume utile de la chambre de pompe 120. La pression à l'intérieur de la chambre augmente jusqu'à ce qu'elle excède la force exercée par le ressort 127. Le clapet d'entrée s'ouvre alors en décollant la surface tronconique 1252 du siège 1225. Un passage est alors libéré pour le produit fluide sous pression à l'intérieur de la chambre de pompe 120 qui peut s'écouler à travers le canal de sortie 123. Le clapet d'entrée est toujours fermé par l'engagement du cordon 1251 en coulissement étanche autour de la tubulure 1215.

Pour actionner la pompe, il est prévu une tête d'actionnement (non représenté), qui peut se présenter sous la forme d'un poussoir formant un conduit de sortie débouchant au niveau d'un orifice de distribution, permettant avantageusement une pulvérisation du produit fluide. L'utilisateur appuie à l'aide d'un ou de plusieurs doigt(s) sur le poussoir pour enfoncer la tige d'actionnement dans le corps et ainsi déclencher le processus décrit ci-dessus. Cette tête d'actionnement n'est pas représentée sur les figures, étant donné que pour le remplissage du distributeur 1, cette tête doit être retirée. L'utilisateur peut très facilement procéder au retrait de la tête d'actionnement en tirant tout simplement dessus. La remise en place de la tête d'actionnement est également très simple puisqu'il suffit d'emmancher en force le poussoir sur l'extrémité supérieure de la tige d'actionnement.

La pompe 12 est engagée et maintenue dans le col 111 du récipient 11 au moyen d'une bague à sertir 13. La bague à sertir vient en prise avec la pompe par exemple au niveau de l'extrémité supérieure du corps 121 en saisissant par exemple la virole 126. Ainsi, la bague à sertir 113 contribue également à la fixation définitive de la virole 126 dans le corps de pompe 121. La bague à sertir 13 est sertie autour du col 111 qui forme avantageusement un rebord saillant vers l'extérieur. Pour garantir l'étanchéité au niveau du col, il est avantageusement prévu un joint de col annulaire 14 qui est appuyé sur l'extrémité supérieure du col par la bague à sertir 13. De cette manière, le réservoir 110 est parfaitement isolé de l'extérieur. Etant donné que la pompe 12 est du type sans reprise d'air, le produit prélevé et distribué par l'actionnement de la pompe a pour effet de diminuer le volume de produit fluide stocké dans le réservoir 110, alors que le volume utile du réservoir reste fixe. Il en résulte que la pression à l'intérieur du réservoir 110 diminue pour atteindre un maximum de dépression lorsque le réservoir est vide de produit fluide. Etant donné que la fixation de la pompe sur le récipient est étanche, cette dépression persiste à l'intérieur du réservoir. Normalement, le distributeur est alors mis au rebut.

L'esprit de la présente invention est d'utiliser cette dépression à l'intérieur du réservoir 110 pour remplir à nouveau le distributeur à travers la pompe 12. Ce procédé peut être mis en oeuvre avec n'importe quel distributeur comprenant un récipient rigide et une pompe « airless » fixée dessus de manière étanche. Le type particulier de pompe qui a été décrit ci-dessus ne constitue qu'un exemple de pompe non limitatif parmi d'autres.

La présente invention met également en oeuvre une source externe qui est désignée ici dans son ensemble par la référence numérique 2. La source externe 2 et le distributeur 1 forme un ensemble de remplissage selon l'invention. La source externe 2 peut se présenter sous des formes très diverses. Elle peut se présenter sous la forme d'une simple alimentation en produit fluide spécialement dédiée au remplissage de distributeur de produit fluide 1. La source externe peut alors être une fontaine de remplissage, par exemple installée dans des magasins de sorte que les utilisateurs peuvent venir recharger leurs distributeurs de produit fluide vides en dépression. La source externe peut également se présenter sous la forme d'un autre distributeur de produit fluide comparable au distributeur 1. C'est le cas de la source externe 2 représentée sur les figures. La source externe est en réalité un distributeur de produit fluide 2 comprenant également un récipient 21 définissant intérieurement un réservoir de produit fluide 210 qui va servir de réserve de remplissage pour le distributeur 1. Le récipient comprend un col 211 dans lequel est monté une pompe 22 ou plus généralement un organe de distribution de produit fluide. La pompe 22 peut être identique, similaire ou encore d'un type totalement différent de celui du distributeur 1. Le col peut aussi être simplement pourvu d'un bouchon. Le volume utile du réservoir 210 peut par exemple être d'une contenance supérieure ou très largement supérieure à celui du réservoir 110. Ainsi, la réserve de produit fluide constitué par le réservoir 210 permet plusieurs remplissages du réservoir 110 du distributeur 1. Le distributeur source 2 comprend un fond 212 équipé d'une unité de remplissage que nous allons maintenant décrire en détail. Cette unité est destinée à coopérer avec le distributeur 1 pour permettre le remplissage de son réservoir vide en dépression.

Le produit fluide stocké dans le réservoir 210 est à une pression sensiblement égale ou inférieure à la pression atmosphérique. En tout cas, la pression n'a pas besoin d'être largement supérieure à la pression atmosphérique.

L'unité comprend un plateau de fond 25 qui est engagé de manière étanche dans le fond 212 du récipient 21. Ce plateau de fond 25 constitue en réalité la paroi de fond du réservoir 210. Ce plateau de fond 25 définit un orifice d'alimentation 251 qui traverse le plateau de fond 25. Par conséquent cet orifice constitue un passage de communication entre l'intérieur du réservoir 210 et l'extérieur. Le plateau 25 support un clapet d'alimentation 26 qui permet d'obturer sélectivement l'orifice d'alimentation 251. Ce clapet d'alimentation 26 comprend un organe mobile de clapet d'alimentation 262 destiné à venir en contact sélectif étanche sur un siège de clapet d'alimentation 261. Le siège 261 peut être formé par le bord périphérique annulaire de l'orifice d'alimentation 251 tourné vers l'intérieur du réservoir 210. L'organe mobile 262 peut être sollicité élastiquement par un ressort 263 sur le siège 261. Ainsi, l'orifice d'alimentation 251 ne peut être alimenté en produit fluide à partir du réservoir 210 que lorsque l'organe mobile 262 est dégagé ou décollé de son siège 261. Du fait que le produit fluide n'est pas sous pression dans le réservoir 210, une ouverture accidentelle du clapet 26 n'entraîne pas de fuite importante. L'organe mobile 262 comprend également une broche 2621 qui s'étend axialement à travers l'orifice d'alimentation 251. Cette broche 2621 comprend une extrémité libre de poussée 2622.

L'unité de remplissage comprend optionnellement, mais avantageusement, des moyens de blocage 27 logés dans un logement formé par le plateau de fond 25. Ces moyens de blocage 27 permettent de bloquer la tige d'actionnement 122 par rapport au corps de pompe 121 et de ce fait par rapport au récipient 11. Le but est d'empêcher que la tige d'actionnement 122 ne se déplace vers l'intérieur du corps lors de l'opération de remplissage. Ces moyens de blocage 27 comprennent ici deux bras articulés 27, mais on peut très bien prévoir encore davantage de bras par exemple 3, 4, 5, 6 ou même plus. Chaque bras est monté pivotant autour d'un axe de pivotement 272 solidaire du plateau de fond 25. Chaque bras 27 comprend d'autre part une mâchoire 271 destinée à venir en prise avec la paroi externe de la tige d'actionnement 122. Les mâchoires des bras 27 forment ensemble un logement interne central 270, dont la taille varie en fonction du pivotement des bras 27. Le logement central 270 peut ainsi se resserrer de sorte que les mâchoires 271 viennent en prise serrante tout autour de la tige d'actionnement 122. Toutefois, en position de repos telle que représentée sur les figures 2a et 2b, le logement central 270 présente une taille ou plus précisément un diamètre interne supérieur au diamètre externe de la tige d'actionnement 122. En revanche, lorsque les bras 27 vont pivoter vers le haut, le diamètre interne du logement central 270 va se réduire de sorte que les mâchoires 271 vont venir en prise serrante avec la tige 122. Selon une autre caractéristique avantageuse, les moyens de blocage 27 comprennent des moyens de came ou de levier qui se présentent ici sous la forme d'une surface d'appui 273 destinées à venir en prise glissante avec un élément fixe du distributeur, qui est ici une partie de la bague à sertir 13. Le système de levier a pour but que les mâchoires 271, une fois qu'elles sont en prises serrante autour de la tige 122, exercent une légère traction vers le haut. En raison de la disposition particulière de la mâchoire 271, de l'axe de pivotement 272 et de la surface d'appui 273, on comprend aisément qu'un pivotement vers le haut du bras a premièrement pour effet d'amener la mâchoire 271 en contact de la tige 122 du fait du resserrement du logement central, mais a également pour effet d'augmenter la distance axiale qui sépare la mâchoire 271 de la surface d'appui 273. En d'autres termes, la mâchoire 271 s'éloigne axialement de la surface d'appui 273 lorsque les bras 27 pivotent vers le haut. Cet éloignement vertical ou axial des mâchoires 271 a pour effet d'exercer une traction vers le haut sur la tige d'actionnement 122.

La structure de l'ensemble de remplissage selon l'invention ayant maintenant été décrite en détail, nous allons maintenant décrire un cycle opératoire complet de remplissage en décrivant toutes les étapes du procédé de remplissage selon l'invention.

Nous nous référerons tout d'abord aux figures 2a et 2b. La tige d'actionnement 122 est déjà engagée entre les mâchoires 271 dans le logement central 270. La broche 2621 est engagée dans le canal de sortie 123. Toutefois, son extrémité de poussée 2622 n'est pas encore en contact du sommet 1253 de la pièce de clapet 125. Les surfaces d'appui 273 sont déjà en contact de la bague à sertir 13. D'autre part, l'extrémité supérieure de la tige d'actionnement 122 est déjà partiellement engagée de manière étanche dans l'orifice d'alimentation 251. Les mâchoires 271 ne sont pas encore en prise serrante autour de la tige 122.

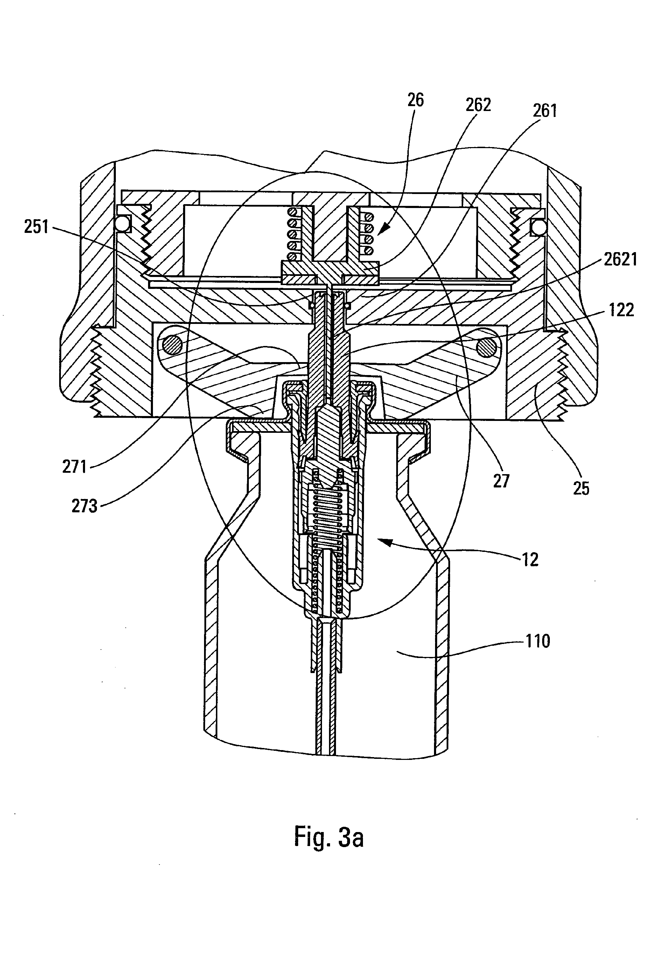

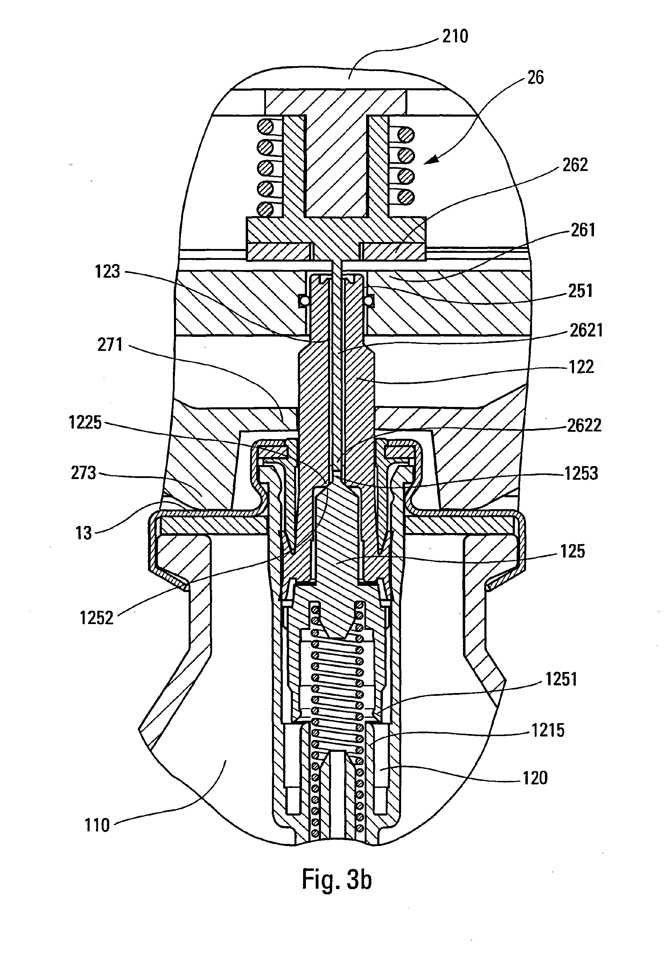

Lorsque l'on continue à appuyer le distributeur 1 vers le distributeur 2, on parvient à la position représentée sur les figures 3a et 3b. Les mâchoires 271 sont maintenant en prise serrante autour de la tige d'actionnement 122. Les surfaces d'appui 273 sont en contact appuyé contre la bague à sertir 13. L'extrémité supérieure de la tige d'actionnement 122 est engagée plus profondément à l'intérieur de l'orifice d'alimentation 251. L'extrémité de poussée 2622 de la broche 2621 est en contact appuyée contre le sommet 1253 de la pièce de clapet 125. L'appui de la broche 2621 sur la pièce de clapet 125 est d'ailleurs suffisant pour décoller l'organe mobile 262 du clapet d'alimentation 26 de son siège de clapet 261. Un passage d'alimentation est ainsi créé qui permet au produit fluide stocké dans le réservoir 210 de parvenir jusqu'à l'intérieur du canal de sortie 123. On rappelle ici que le produit fluide stocké dans le réservoir d'alimentation 210 est à une pression sensiblement égale ou inférieure à la pression atmosphérique. De ce fait, le produit fluide peut s'écouler dans le canal de sortie 123 sensiblement sans pression. A ce stade, il faut noter que le clapet de sortie 1225, 1252 est fermé. L'ouverture du clapet d'alimentation 26, alors que le clapet de sortie 1225, 1252 est fermé, est possible en calibrant le ressort du clapet d'alimentation 26 à une valeur inférieure au ressort de rappel 127 de la pompe 12. Ainsi, c'est le ressort du clapet d'alimentation 26 qui va être comprimé en premier et ainsi permettre l'ouverture préalable du clapet d'alimentation 26 avant l'ouverture du clapet de sortie 1225, 1252.

En exerçant alors une pression encore supérieure sur le distributeur 1 vers le distributeur source 2, l'extrémité supérieure de la tige d'actionnement 122 va pénétrer encore plus profondément dans l'ouverture d'alimentation 251. Etant donné que le clapet d'alimentation 26 était déjà ouvert, cette pression supplémentaire a pour effet de repousser la pièce de clapet 125 de manière à ouvrir le clapet de sortie. Une communication de fluide est ainsi établie entre les deux réservoirs 210 et 110. En effet, non seulement le clapet d'alimentation 26 et le clapet de sortie sont ouverts, mais le clapet d'entrée 1251, 1215 est également ouvert. La forte dépression régnant dans le réservoir 110 est mis en présence du produit fluide du réservoir source 210 qui est à une pression sensiblement égale ou légèrement inférieure à la pression atmosphérique. Il en résulte que le produit fluide du réservoir source 210, qui était déjà présent au niveau du canal de sortie 123 du fait de l'ouverture préalable du clapet d'alimentation 26, va être aspiré dans le réservoir 110 à travers la pompe 12, ou plus précisément à travers le canal de sortie 123, le clapet de sortie ouvert, la chambre de pompe 120, le clapet d'entrée ouvert et l'entrée de la pompe éventuellement pourvue d'un tube plongeur. L'aspiration du produit fluide remplit ainsi le réservoir 110 jusqu'à ce que les pressions à l'intérieur des deux réservoirs 110 et 210 s'équilibrent. En choisissant un réservoir source 210 d'une contenance largement supérieure à celle du réservoir à remplir 110, cela permet un remplissage complet du réservoir 110. Il faut également noter que les mâchoires 271, en prise avec la tige 122 ont effectué lors de cette dernière étape de remplissage un léger déplacement vers le haut ce qui a engendré une légère traction sur la tige d'actionnement 122. De toute façon, même sans légère traction, il est préférable de maintenir la tige d'actionnement 22 en place, c'est-à-dire de manière fixe ou statique par rapport au corps 121. En effet, en raison de la forte dépression qui règne à l'intérieur du réservoir 110, la tige d'actionnement 122 à tendance à être aspirée dans le corps lorsque le clapet de sortie est ouvert par la broche 2621. Ceci aurait pour fâcheuse conséquence d'obturer immédiatement le clapet d'entrée, ce qui rendrait alors le remplissage impossible. Le blocage de la tige d'actionnement 122 a donc pour but de maintenir le clapet d'entrée ouvert. Une légère traction optionnelle vers le haut a pour conséquence avantageuse d'augmenter les sections de passage pour le produit fluide en provenance du réservoir source 210. Ainsi les pertes de charge sont plus faibles et le remplissage plus rapide et plus complet.

Une fois l'opération de remplissage terminée, il suffit de retirer la tige d'actionnement de la broche 2621 et d'entre les mâchoires 271. Le retrait opère dans un premier temps la fermeture du clapet de sortie de la pompe 12 puis dans un deuxième temps la fermeture du clapet d'alimentation 26. Ainsi, les deux réservoirs 110 et 210 sont à nouveau isolés de l'extérieur. L'utilisateur n'a plus qu'à remettre la tête d'actionnement en place sur l'extrémité supérieure de la tige d'actionnement. Après vidage du réservoir 110 par actionnement de la pompe 12, le distributeur 1 peut à nouveau être rempli de la manière décrite ci-dessus en utilisant une source externe 2 dans laquelle le produit fluide stocké à l'intérieur du réservoir source n'est pas sous pression.

## Revendications

1. Procédé de remplissage de réservoir (110) de distributeur de produit fluide (1) comprenant :
- un réservoir (110) destiné à contenir un produit fluide à une pression sensiblement égale ou inférieure à la pression atmosphérique,
- une pompe (12) associée de manière étanche au réservoir pour prélever du produit fluide dans le réservoir, la pompe étant du type sans reprise d'air de sorte que la pression à l'intérieur du réservoir diminue à mesure que la pompe prélève du produit fluide, ladite pompe comprenant un clapet d'entrée (1215, 1251) et un clapet de sortie (1225, 1252), le clapet d'entrée communiquant avec le réservoir et le clapet de sortie communiquant avec un canal de sortie (123), le clapet d'entrée communiquant avec le clapet de sortie à travers une chambre de pompe (120),
ledit procédé comprenant :
- une étape préalable de vidage du réservoir par prélèvement du produit fluide au moyen de la pompe, de sorte qu'une dépression est créée dans le réservoir,
- une étape d'alimentation externe en produit fluide consistant à alimenter le canal de sortie (123) avec du produit fluide issu d'une source externe (2), et
- une étape d'ouverture consistant à ouvrir le clapet de sortie (1225, 1252), en maintenant le clapet d'entrée (1215, 1251) ouvert, de sorte que la dépression régnant dans le réservoir (110) aspire du produit fluide issu de la source (2) à travers le canal de sortie, le clapet de sortie ouvert, la chambre de pompe et le clapet d'entrée ouvert jusque dans le réservoir.

2. Procédé de remplissage selon la revendication 1, dans lequel le produit fluide issu de la source externe (2) est alimenté au canal de sortie (123) à une pression sensiblement égale ou inférieure à la pression atmosphérique.

3. Source externe de remplissage de produit fluide pour un distributeur de produit fluide (1) comprenant :
■ un réservoir (110) destiné à contenir un produit fluide à une pression sensiblement égale ou inférieure à la pression atmosphérique,
■ une pompe (12) associée de manière étanche au réservoir (110) pour prélever du produit fluide dans le réservoir, la pompe étant du type sans reprise d'air de sorte que la pression à l'intérieur du réservoir diminue à mesure que la pompe prélève du produit fluide, ladite pompe comprenant un clapet d'entrée (1215, 1251) et un clapet de sortie (1225, 1252), le clapet d'entrée communiquant avec le réservoir et le clapet de sortie communiquant avec un canal de sortie (123), le clapet d'entrée communiquant avec le clapet de sortie à travers une chambre de pompe (120),
la source externe étant **caractérisée en ce qu'**elle comprend :
■ une réserve de produit fluide (210),
■ un orifice d'alimentation en produit fluide (251) destiné à être connecté au canal de sortie (123) du distributeur (1),
■ un clapet d'alimentation (26) disposé entre la réserve (210) et l'orifice d'alimentation (251), ledit clapet d'alimentation étant sollicité en position ouverte lorsque l'orifice d'alimentation est connecté au canal de sortie du distributeur, ledit clapet d'alimentation comprenant un organe mobile (262) en appui étanche sélectif sur un siège de clapet (261).

4. Source selon la revendication 3, dans lequel le produit fluide contenu dans la réserve (210) de la source (2) est à une pression sensiblement égale ou légèrement inférieure à la pression atmosphérique, de sorte que le produit fluide issu de l'orifice d'alimentation (251) est aspiré dans le réservoir (110) du distributeur (1) par la dépression qui y règne.

5. Source selon la revendication 3 ou 4, comprenant des moyens d'ouverture (2621) aptes à ouvrir le clapet de sortie (1225, 1252) du distributeur (1).

6. Source selon la revendication 5, dans lequel les moyens d'ouverture (2621) coopèrent également avec le clapet d'alimentation (26) pour l'ouvrir avant d'ouvrir le clapet de sortie.

7. Source selon la revendication 6, dans lequel les moyens d'ouverture comprennent une broche (2621) définissant une extrémité de liaison solidaire de l'organe mobile (262) et une extrémité libre de poussée (2622) destinée à être engagée dans le canal de sortie (123) du distributeur pour ouvrir le clapet de sortie (1225, 1252).

8. Source selon l'une quelconque des revendications 3 à 7, le canal de sortie (123) du distributeur étant formé par une tige d'actionnement (122) déplaçable axialement dans un corps de pompe (121), la tige supportant un piston (124) apte à faire varier le volume utile de la chambre de pompe (120), dans lequel il est prévu des moyens de blocage (27) pour bloquer la tige d'actionnement (122) par rapport au corps de pompe (121).

9. Source selon la revendication 8, dans lequel les moyens de blocage (27) comprennent au moins deux mâchoires articulées (271) entre lesquelles la tige d'actionnement (122) est destinée à être maintenue de manière serrante.

10. Source selon la revendication 9, dans lequel les moyens de blocage (27) comprennent un système de levier (273) apte à exercer une traction qui sollicite la tige (122) hors du corps (121) alors qu'elle est déjà maintenue par les mâchoires.

11. Source selon la revendication 9, dans lequel les moyens de blocage comprennent au moins deux bras articulés (27) présentant chacun une extrémité fixe (273) et une extrémité libre formant une mâchoire (271), les mâchoires étant disposées de manière à définir un logement central (270) de taille variable à mesure que les mâchoires se déplacent par pivotement des bras, les bras formant en outre chacun une surface d'appui destinée à venir en contact d'appui (273) sur un élément fixe (13) du distributeur, un pivotement des bras dans le sens d'une diminution de la taille du logement central, alors que la tige (122) est insérée dans ce logement (270), amène les mâchoires en prise serrante avec la tige et les surfaces d'appui en contact de l'élément fixe de manière à exercer une traction sur la tige hors du corps par l'intermédiaire des mâchoires.

12. Source selon la revendication 9, 10 ou 11, dans lequel l'organe mobile (262) du clapet d'alimentation (26) comprend une broche (2621) destinée à être insérée dans le canal de sortie (123) pour ouvrir le clapet de sortie (1225, 1252), la broche s'étendant de manière centrale et axiale entre les mâchoires (271) des moyens de blocage (27) pour guider axialement le canal de sortie autour de la broche.

13. Source selon l'une quelconque des revendications précédentes, dans lequel la source externe est constituée par un autre distributeur de produit fluide (2) comprenant également un réservoir (210)et une pompe (22).

14. Source selon l'une quelconque des revendications 3 à 13, dans lequel le clapet d'entrée comprend un siège de clapet (1215) et un organe mobile (1251), le clapet de sortie comprend un siège de clapet (1223) et un organe mobile (1252), les organes mobiles étant formés par une pièce de clapet (125) de sorte que les organes mobiles sont solidaires en déplacement, les clapets d'entrée et de sortie pouvant ainsi être ouverts simultanément.

15. Ensemble de distribution et de remplissage comprenant :
- un distributeur de produit fluide comprenant :
■ un réservoir destiné à contenir un produit fluide à une pression sensiblement égale ou inférieure à la pression atmosphérique,
■ une pompe associée de manière étanche au réservoir pour prélever du produit fluide dans le réservoir, la pompe étant du type sans reprise d'air de sorte que la pression à l'intérieur du réservoir diminue à mesure que la pompe prélève du produit fluide, ladite pompe comprenant un clapet d'entrée et un clapet de sortie, le clapet d'entrée communiquant avec le réservoir et le clapet de sortie communiquant avec un canal de sortie, le clapet d'entrée communiquant avec le clapet de sortie à travers une chambre de pompe, et
- une source externe de remplissage (2) selon l'une quelconque des revendications 3 à 14.
